(19) Europäisches Patentamt
European Patent Office
Office européen des brevets

(11) Veröffentlichungsnummer: **0 058 635**
**B1**

# (12) EUROPÄISCHE PATENTSCHRIFT

(45) Veröffentlichungstag der Patentschrift: **03.07.85**

(21) Anmeldenummer: **82810043.8**

(22) Anmeldetag: **03.02.82**

(51) Int. Cl.⁴: **C 07 D 233/24**, C 07 D 401/12, C 07 F 9/65, A 01 N 43/50, A 01 N 57/32

(54) **Substituierte 2-(Anilinomethyl)-2-imidazolin-Derivate, Verfahren zu ihrer Herstellung, Mittel, welche diese Derivate enthalten, und ihre Verwendung zur Bekämpfung von Schädlingen.**

(30) Priorität: **09.02.81 CH 842/81**
**09.02.81 CH 843/81**

(43) Veröffentlichungstag der Anmeldung: **25.08.82 Patentblatt 82/34**

(45) Bekanntmachung des Hinweises auf die Patenterteilung: **03.07.85 Patentblatt 85/27**

(84) Benannte Vertragsstaaten: **AT BE CH DE FR IT LI NL**

(56) Entgegenhaltungen:
**EP - A - 0 001 647**
**EP - A - 0 008 071**
**EP - A - 0 010 754**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Böger, Manfred, Wilhem Glock-Strasse 14, D-7858 Weil am Rhein 5 (DE)**
Erfinder: **Bürckhardt, Urs, Dr., Rittergasse 29, CH-4051 Basel (CH)**
Erfinder: **Kristinsson, Haukur, Dr., Kreuzackerweg 19, CH-4103 Bottmingen (CH)**
Erfinder: **Mattern, Günter, Dr., Sichternstrasse 35, CH-4410 Liestal (CH)**
Erfinder: **Traber, Walter, Dr., Neueneichweg 12, CH-4153 Reinach (CH)**

**EP 0 058 635 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue substituierte 2-(Anilinomethyl)-2-imidazolin-Derivate, Verfahren zu ihrer Herstellung, Schädlingsbekämpfungsmittel, welche die vorstehend genannten Derivate als aktive Komponente enthalten, und Verfahren zur Bekämpfung von Schädlingen unter Verwendung der neuen Verbindungen.

2-(Anilinoalkyl)-2-imidazolin-Derivate mit pestizider, vor allem ektoparasitizider Wirkung sind bekannt (siehe z.B. britische Patentschrift 2 023 603, europäische Patentschrift 0 001 647, DE-OS 2 756 638 und DE-OS 2 756 639). Ferner sind bekannt Phenoxy-(alkyl)-imidazoline mit pestizider, vor allem akarizider Wirksamkeit (vgl. EP-8071 und EP-10754). Die bekannten Wirkstoffe können die an sie gestellten Forderungen jedoch nicht in dem gewünschten Masse voll befriedigen. Mit der vorliegenden Erfindung werden neuartige Verbindungen dieses Typs bereitgestellt, die eine ausgeprägte Wirkung gegen Schädlinge, insbesondere gegen Vertreter der Ordnung Acarina, aufweisen, und welche aufgrund ihrer vorteilhaften biologischen Eigenschaften für die praktische Anwendung besonders geeignet sind.

Die erfindungsgemässen neuen substituierten 2-(Anilinomethyl)-2-imidazolin-Derivate entsprechend der Formel I

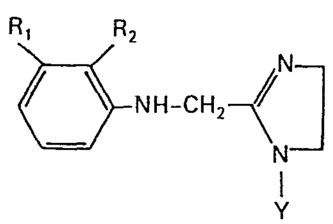

(I)

in welcher bedeuten:

$R_1$ und $R_2$ unabhängig voneinander ein Chloratom oder die Methylgruppe,

Y die Reste 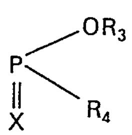 oder $-CH=N-R_5$,

worin darstellen:

$R_3$ Methyl oder Äthyl,

$R_4$ Alkyl mit 1 bis 4 Kohlenstoffatomen, Alkoxy mit 1 bis 2 Kohlenstoffatomen, Alkylthio mit 1 bis 4 Kohlenstoffatomen oder Phenyl,

X ein Sauerstoffatom oder ein Schwefelatom und

$R_5$ einen mit einem Kohlenstoffatom an den Rest des Moleküls gebundenen unsubstituierten oder substituierten Pyridinylrest, dessen Substituenten aus der Gruppe Halogen und Alkyl mit 1 bis 4 Kohlenstoffatomen ausgewählt sind, einschliesslich ihrer Säureadditionssalze.

Formal umfassen die Verbindungen der Formel I phosphorhaltige Derivate

(Y = 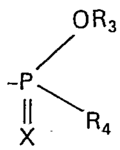 )

und Pyridinylimin-Derivate ($Y = -CH=N-R_5$).

Die für $R_4$ in Frage kommenden Alkyl- und Alkylthiogruppen sind die Methyl-, Äthyl-, n-Propyl-, iso-Propyl-, n-Butyl-, iso-Butyl-, sek. Butyl- und tert. Butylgruppe sowie die Methylthio-, Äthylthio-, n-Propylthio-, iso-Propylthio-, n-Butylthio-, iso-Butylthio, sek. Butylthio- und tert. Butylthiogruppe.

In $R_5$ als Substituenten des Pyridinylrestes sind unter Alkylgruppen die Methyl-, Äthyl-, n-Propyl-, iso-Propyl-, n-Butyl-, iso-Butyl, sek.-Butyl und tert.-Butylgruppe und unter Halogen Fluor, Chlor, Brom und Jod, vorzugsweise Chlor oder Brom, zu verstehen.

Die Verbindungen der Formel I liegen als freie Basen oder als Säureadditionssalze, z.B. Mineralsalze, vor und können erfindungsgemäss in beiden Formen verwendet werden.

Es hat sich nun überraschenderweise gezeigt, dass die erfindungsgemässen Verbindungen der Formel I eine ausgeprägte Wirkung sowohl gegen pflanzenschädigende Vertreter der Ordnung Acarina (z.B. Milben der Familien Tarsonemidae, Erophyidae, Tyroglyphidae, Glycyphagidae und insbesondere Tetranychidae) als auch gegen ektoparasitäre Vertreter dieser Ordnung (z.B. Milben und Zecken der Familien Sarcoptidae, Dermanyssidae, Ixodidae, und Argasidae), welche an Nutztieren erhebliche Schäden verursachen können, und gegen Vertreter der Ordnungen Phthiraptera (Tierläuse) und Phytophthires (Pflanzenläuse) sowie gegen Dipteren, welche zur Familie Calliphoridae gehören, aufweisen. Als Vertreter der Familie Calliphoridae ist vor allem die Gattung Lucilia, darunter insbesondere die Species Lucilia sericata (blowfly) zu erwähnen.

Die erfindungsgemässen Verbindungen besitzen die Fähigkeit, bei der Applikation an Pflanzen durch die Blattoberfläche in das Innere der Blätter zu penetrieren. Die Verbindungen der Formel I einschliesslich ihrer für Warmblüter nicht-toxischen Säureadditionssalze eignen sich daher besonders zur Bekämpfung saugender Pflanzenschädlinge, insbesondere pflanzenparasitärer Vertreter der Ordnung Acaraina, in Kulturen von Nutz- und Zierpflanzen, insbesondere in Obst- und Gemüsekulturen und vor allem auf dem Gebiet des Citrusanbaus.

Auf dem tierparasitären Sektor ist vor allem die Bekämpfung ektoparasitärer Milben bei Nutztieren hervorzuheben, wobei die Zecken als Untergruppe der Milben besonders in Betracht zu ziehen sind.

Die Wirksamkeit gegen Schädlinge ist mit einer für die praktische Anwendung günstigen Toxizität gegenüber Warmblütern gekoppelt, wodurch sich die Verbindungen der Formel I sowie deren für Warmblüter nicht-toxische Säureadditionssalze besonders zur Bekämpfung von Schädlingen der Ordnung Acarina in Kulturen von Nutz- und Zierpflanzen sowie zur Bekämpfung von ektoparasitären Zecken und Milben bei Nutztieren eignen.

Unter den Verbindungen der Formel I sind in der Reihe der Phosphorester solche Verbindungen einschliesslich ihrer Säureadditionssalze bevorzugt, in denen X Sauerstoff, $R_3$ Äthyl und $R_4$ Alkylthio mit 3 oder 4 Kohlenstoffatomen darstellen und $R_1$ und $R_2$ die unter Formel I angegebenen Bedeutungen besitzen. Unter den Formamidinen der Formel I sind hingegen diejenigen Verbindungen einschliesslich ihrer Säureadditionssalze bevorzugt, bei denen $R_5$ ein in 2-Stellung an den Rest des Moleküls gebundenen unsubstituierten oder methyl-substituierten Pyridinylrest darstellt und $R_1$ und $R_2$ die unter Formel I angegebenen Bedeutungen besitzen.

Folgende Verbindungen einschliesslich ihrer Säureadditionssalze sind in ihrer Wirkung als besonders bevorzugt anzusehen:
1-(O-Äthyl-S-sek.butyl-thiophosphonyl-2-(2′,3′-dimethylanilinomethyl)-2-imidazolin,
1-(O-Äthyl-S-isobutyl-thiophosphonyl-2-(2′,3′-dimethylanilinomethyl)-2-imidazolin,
1-(O-Äthyl-S-n-propyl-thiophosphonyl-2-(2′,3′-dimethylanilinomethyl)-2-imidazolin,
1-(O-Äthyl-äthyl-thiophosphinyl-2-(2′,3′-dimethylanilinomethyl)-2-imidazolin,
1-(O-Äthyl-S-isobutylthiophosphonyl-2-(2′,3′-dichloranilinomethyl)-2-imidazolin,
1-(O-Äthyl-S-n-propylthiophosphonyl-2-(2′,3′-dichloranilinomethyl)-2-imidazolin,
1-(O-Äthyl-S-n-propyl-thiophosphonyl-2-(2′-methyl-3′-chloranilinomethyl)-2-imidazolin,
1-(O-Äthyl-S-sek.butyl-thiophosphonyl-2-(2′-methyl-3′-chloranilinomethyl)-2-imidazolin,
N-([2-(2,3-Dimethylanilinomethyl)-2-imidazolin-1-yl]-methyliden)-N-pyridin-2-yl)imin,
N-([2-(2,3-Dichloranilinomethyl)-2-imidazolin-1-yl]-methyliden)-N-(pyridin-2-yl)imin,
N-([2-(2,3-Dichloranilinomethyl)-2-imidazolin-1-yl]-methyliden)-N-(3-methylpyridin-2-yl)imin,
N-([2-(2,3-Dichloranilinomethyl)-2-imidazolin-1-yl]-methyliden)-N-(6-methylpyridin-2-yl)imin
und
N([2-(2,3-Dichloranilinomethyl)-2-imidazolin-1-yl]-methyliden)-N-(4-methylpyridin-2-yl)imin.

Die Verbindungen der Formel I werden analog bekannten Verfahren hergestellt, indem man z.B. eine Verbindung der Formel II

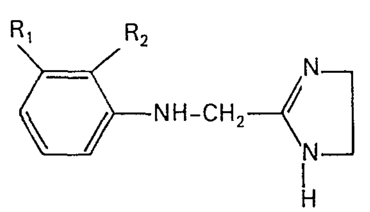

(II)

umsetzt mit:

a) einer Verbindung der Formel IIIa

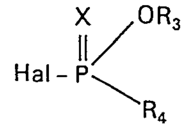

(IIIa)

wobei in den Formeln II und IIIa, $R_1$, $R_2$ $R_3$, $R_4$ und X die für Formel I angegebenen Bedeutungen haben und Hal für ein Halogenatom, insbesondere ein Chlor- oder Bromatom steht, bei einer Temperatur zwischen -20° und 80°C bei normalem oder leicht erhöhtem Druck in Gegenwart einer Base und vorzugsweise in einem gegenüber den Reaktionsteilnehmern inerten Lösungs- oder Verdünnungsmittel und

b) einer Verbindung der Formel IIIb

$$R_6\text{-O-CH=N-}R_5 \qquad \text{(IIIb)}$$

wobei in den Formeln II und IIIb $R_1$, $R_2$ und $R_5$ die für Formel I angegebenen Bedeutungen besitzen und $R_6$ Methyl oder Äthyl darstellt, bei einer Temperatur zwischen -20° und 120°C bei normalem oder leicht erhöhtem Druck und vorzugsweise in einem gegenüber den Reaktionsteilnehmern inerten Lösungs- oder Verdünnungsmittel.

Zur Verwendung in den vorstehend beschriebenen Reaktionen eignen sich als Lösungs- oder Verdünnungsmittel z.B. Äther und ätherartige Verbindungen wie Diäthyläther, Di-isopropyläther, Dioxan und Tetrahydrofuran; aromatische Kohlenwasserstoffe wie Benzol, Toluol und Xylole; und Ketone wie Aceton, Methyläthylketon und Cyclohexanon.

Als geeignete Basen kommen insbesondere tertiäre Amine, wie Trialkylamine, Pyridine und Dialkylaniline, ferner Hydroxide, Oxide, Carbonate und Bicarbonate von Alkali- und Erdalkalimetallen sowie Alkalimetallalkoholate wie z.B. Kalium-tert.butylat und Natriummethylat in Betracht.

Die auf diese Weise hergestellten Verbindungen der Formel I können nach an sich bekannten Methoden in ihre Säuresalze überführt werden.

Die in den vorstehend aufgeführten Reaktionen verwendeten Ausgangsstoffe sind bekannt (vgl. DE-OS 2 750 902 und DE-OS 2 756 638) bzw. können analog bekannten Methoden hergestellt werden.

Beispiel 1

Herstellung von 1-(O-Äthyl-S-n-propyl-thiophosphonyl)-2-(2′,3′-dimethylanilinomethyl)-2-imidazolin

Zu einer Suspension von 8,1 g 2-(2′,3′-Dimethylanilinomethyl)-2-imidazolin in 100 ml Toluol werden bie 0° bis 10°C 4,3 g Triäthylamin zugegeben und anschliessend bei 0° bis 16°C langsam eine Lösung von 8,1 g O-Äthyl-S-n-propyl-thiochlorphosphat in 50 ml Toluol unter ständigem Rühren zugetropft. Das erhaltene Reaktionsgemisch wird eine Stunde

bei ca. 10°C gerührt, mit 100 ml Wasser versetzt und die Toluolphase noch mehrmals mit wenig Wasser gewaschen. Nach dem Abdampfen der vorher getrockneten Toluollösung und Trocknen am Hochvacuum bei 50° erhält man die Verbindung der Formel

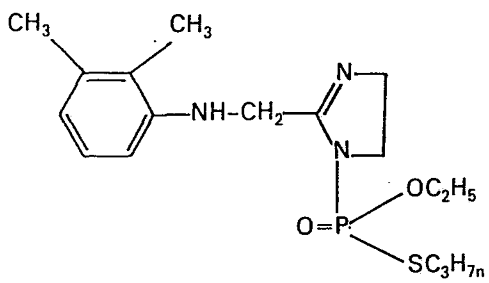

als klares gelbes Öl mit einer Refraktion von $n_D^{20}$ 1,5601.

Beispiel 2

N-([2-(2,3-Dimethylanilinomethyl)-2-imidazolin-1-yl]-methyliden)-N-(3-methylpyridin-2-yl) imin

8,0 g 2-(2,3-Dimethylanilinomethyl)-2-imidazolin und 6,6 g N-(3-Methylpyridin-2-yl)formiminoäthyläther der Formel IV

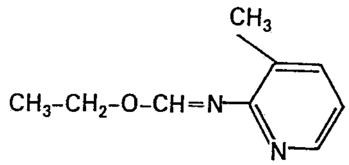

(IV)

werden mit 100 ml Toluol 5 Stunden bei einer Temperatur von 70°C gerührt. Das erhaltene Reaktionsgemisch wird eingedampft und der Rückstand zweimal aus Toluol umkristallisiert. Man erhält N-([2-(2,3-Dimethylanilinomethyl)-2-imidazolin-1-yl-methyliden)-N-(3-methylpyridin-2-yl)amin der Formel V

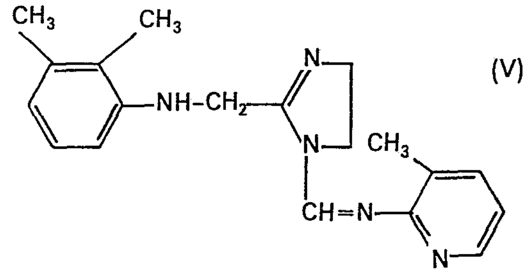

(V)

als beigefarbenes Pulver; Smp. 176–178°C.

Auf analoge Weise werden die folgenden Verbindungen der Formel I hergestellt:

Tabelle Ia

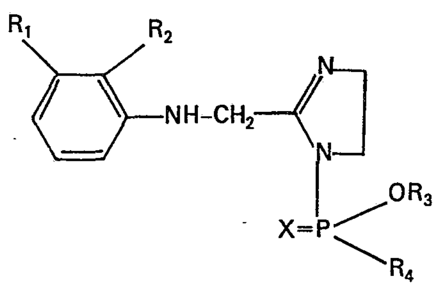

| Nr. | $R_1$ | $R_2$ | $R_3$ | $R_4$ | X | Physikal. Daten |
|---|---|---|---|---|---|---|
| 1a | $CH_3$ | $CH_3$ | $C_2H_5$ | 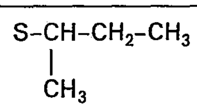 | 0 | $n_D^{20}$ 1,5510 |
| 2a | $CH_3$ | $CH_3$ | $C_2H_5$ | 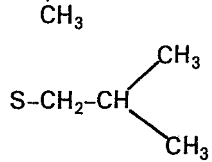 | O | $n_D^{20}$ 1,5516 |
| 3a | $CH_3$ | $CH_3$ | $C_2H_5$ | $S-CH_2-CH_2-CH_3$ | S | Smp. 59–61°C |
| 4a | $CH_3$ | $CH_3$ | $C_2H_5$ | $S-CH_2-CH_2-CH_3$ | O | $n_D^{20}$ 1,5601 |
| 5a | $CH_3$ | $CH_3$ | $C_2H_5$ | $C_2H_5$ | S | hochviskos |
| 6a | $CH_3$ | $CH_3$ | $C_2H_5$ | $O-C_2H_5$ | O | $n_D^{20}$ 1,5332 |
| 7a | $CH_3$ | $CH_3$ | $C_2H_5$ | $C_6H_5$ | S | $n_D^{20}$ 1,6073 |
| 8a | $CH_3$ | $CH_3$ | $CH_3$ | $O-CH_3$ | O | $n_D^{20}$ 1,5440 |
| 9a | Cl | Cl | $C_2H_5$ | 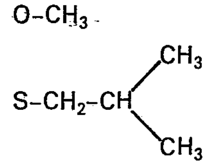 | O | $n_D^{20}$ 1,5656 |

Tabelle Ia (Fortsetzung)

| Nr. | $R_1$ | $R_2$ | $R_3$ | $R_4$ | X | Physikal. Daten |
|---|---|---|---|---|---|---|
| 10a | Cl | Cl | $C_2H_5$ | $S-CH-CH_2-CH_3$<br>\|<br>$CH_3$ | O | $n_D^{20}$ 1,5639 |
| 11a | Cl | Cl | $C_2H_5$ | $S-CH_2-CH_2-CH_3$ | O | $n_D^{20}$ 1,5743 |
| 12a | Cl | Cl | $C_2H_5$ | $S-CH_2-CH_2-CH_3$ | S | $n_D^{20}$ 1,6010 |
| 13a | Cl | Cl | $C_2H_5$ | $O-C_2H_5$ | O | $n_D^{20}$ 1,5505 |
| 14a | Cl | Cl | $CH_3$ | $O-CH_3$ | O | Smp. 92–94°C |
| 15a | Cl | Cl | $C_2H_5$ | $C_6H_5$ | S | $n_D^{20}$ 1,6230 |
| 16a | Cl | $CH_3$ | $C_2H_5$ | $S-CH_2-CH_2-CH_3$ | O | $n_D^{20}$ 1,5674 |
| 17a | Cl | $CH_3$ | $C_2H_5$ | $S-CH-CH_2-CH_3$<br>\|<br>$CH_3$ | O | $n_D^{20}$ 1,5615 |

Tabelle Ib

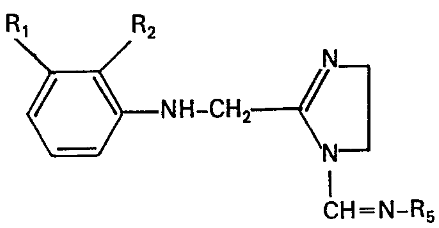

| Nr. | $R_1$ | $R_2$ | $R_5$ | Smp. °C |
|---|---|---|---|---|
| 1b | $CH_3$ | $CH_3$ | 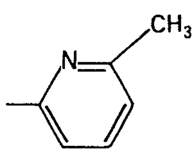 | 173-175 |
| 2b | $CH_3$ | $CH_3$ | 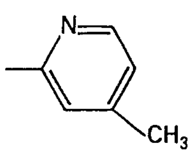 | 193–195 |
| 3b | $CH_3$ | $CH_3$ | 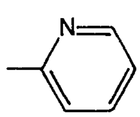 | 194–196 |
| 4b | $CH_3$ | $CH_3$ | 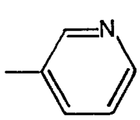 | 181–183 |
| 5b | $CH_3$ | $CH_3$ | 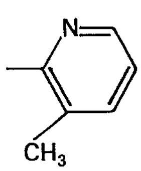 | 176–178 |

Tabelle Ib (Fortsetzung)

| | | | | |
|---|---|---|---|---|
| 6b | Cl | Cl | 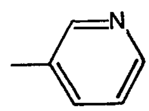 | 172–174 |
| 7b | Cl | Cl | 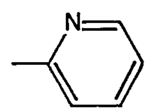 | 193–195 (Z) |
| 8b | Cl | Cl | 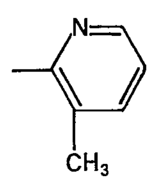 | 175–177 |
| 9b | Cl | Cl | 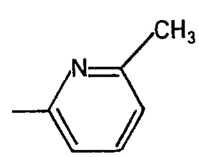 | 181–183 |
| 10b | Cl | Cl | 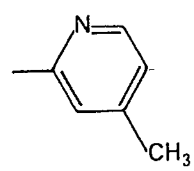 | 202–204 |
| 11b | Cl | $CH_3$ | 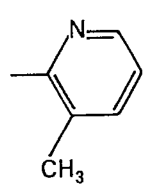 | 181–182 |
| 12b | Cl | $CH_3$ | 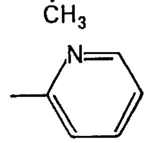 | 181–183 |

Die Verbindungen der Formel I werden erfindungsgemäss als solche verwendet oder bilden einen Bestandteil von Mitteln, welche ausserdem geeignete Trägerstoffe oder Zuschlagstoffe oder Gemische solcher Stoffe enthalten.

Geeignete Träger- und Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Binde- und/oder Düngemitteln.

Die akarizide Wirkung der erfindungsgemässen Mittel lässt sich durch Zusatz anderer Akarizide und/oder Insektizide verbreitern.

Als Zusätze eignen sich z.B.: organische Phosphorverbindungen; Nitrophenole; Formamidine; Harnstoffe; pyrethrinartige Verbindungen; Carbamate und chlorierte Kohlenwasserstoffe.

Als Vertreter der als Zusätze zu den erfindungsgemässen Mitteln genannten Substanzklassen sind beispielsweise folgende Verbindungen zu verstehen:

O-(2-Chlor-1-(2,4-dichlorphenyl)-vinyl)-O,O-diäthylphosphat,
O,O-Diäthyl-O-(2-isopropyl-6-methyl-pyrimidin-4-yl)-phosphothionat,
cis-O,O-Dimethyl-O-(2-dimethylcarbamoyl-1-methylvinyl)-phosphat,
N′-(4-Chlor-o-toluol)-N,N-dimethylformamidin,
N-Methyl-bis(2,4-xylyliminomethyl)-amin,
N-(2-Methyl-4-chlorphenyl)-N′,N′-dimethylthioharnstoff,
2-(2′,4′-Dimethylphenylimino)-3-methyl-thiazolin,
3-Methyl-5-isopropylphenyl-N-methyl-N-n-butanoylcarbamat,
2-Isopropoxyphenyl-N-methylcarbamat,
(+)-3-(2,2-Dichlorvinyl)-2,2-dimethyl-cyclopropancarbonsäure-(α-cyano-3-phenoxybenzyl)-ester,
(S)-α-Cyano-3-phenoxybenzyl(1R)-cis-3-(2,2-dibromvinyl-2,2-dimethyl-cyclopropancarbonsäureester,
(1R)-cis-3-(2,2-Dibromvinyl)-2,2-dimethyl-cyclopropancarbonsäure-(S)-(α-cyano-3-phenoxybenzyl)ester sowie die Verbindung der Formel

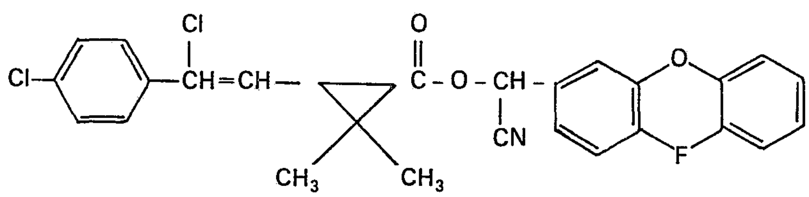

Die erfindungsgemässen Mittel können z.B. als Stäubemittel, Granulate, Dispersionen, Lösungen und Aufschlämmungen sowie als in Wasser dispergierbare Spritzpulver, Pasten, Emulsionen und Emulsionskonzentrate vorliegen und angewendet werden.

Der Gehalt an Wirkstoff (Verbindung der Formel I) in den oben beschriebenen Mitteln liegt zwischen 0,1 bis 95%, dabei ist zu erwähnen, dass bei der Applikation mittels geeigneter Applikationsgeräte auch höhere Konzentrationen eingesetzt werden können.

Die Wirkstoffe der Formel I können beispielsweise wie folgt formuliert werden:

Emulsionskonzentrat I
20 Gew.-Teile Wirkstoff werden in
70 Gew.-Teilen Xylol gelöst und mit
10 Gew.-Teilen eines Emulgiermittels, bestehend aus einem Gemisch eines Arylphenolpolyglykoläthers und dem Calciumsalz der Dodecylbenzolsulfonsäure, versetzt. Das Emulsionskonzentrat kann in beliebigem Verhältnis mit Wasser versetzt werden und bildet dabei eine milchige Emulsion.

Emulsionskonzentrat II
5 bis max. 30 Gew.-Teile Wirkstoff werden unter Rühren bei Zimmertemperatur in
30 Gew.-Teilen Dibutylphthalat,
10 Gew.-Teilen Solven 200 (niederviskoses hocharomat. Erdöldestillat)
10 bis 35 Gew.-Teilen Dutrex 238 FC (viskoses hocharomat. Erdöldestillat) gelöst und mit
10 Gew.-Teilen eines Emulgatorgemisches, bestehend aus Ricinusölpolyglykoläther und dem Calciumsalz der Dodecylbenzolsulfonsäure, versetzt. Das so erhaltene Emulsionskonzentrat gibt in Wasser milchige Emulsionen.

Spritzpulver
5 bis 30 Gew.-Teile Wirkstoff werden in einer Mischapparatur mit
5 Gew.-Teilen eines aufsaugenden Trägermaterials (feindisperse Kieselsäure) und
55 bis 80 Gew.-Teilen eines Trägermaterials (Bolus alba oder Kaolin und einem Dispergiergemisch, bestehend aus
5 Gew.-Teilen eines Na-lauryl-sulfonates und
5 Gew.-Teilen eines Alkyl-aryl-polyglykoläther, intensiv vermischt. Diese Mischung wird auf einer Stift- oder Luftstrahlmühle bis auf 5–15 μm gemahlen. Das so erhaltene Spritzpulver gibt in Wasser eine gute Suspension.

Stäubemittel
5 Gew.-Teile feingemahlener Wirkstoff werden mit
2 Gew.-Teilen einer gefällten Kieselsäure und
93 Gew.-Teilen Talk intensiv gemischt.

«Pour-on»-Lösung

| | |
|---|---|
| Wirkstoff | 30,0 g |
| Natrium-dioctylsulfosuccinat | 3,0 g |
| Benzylalkohol | 48,0 g |
| Erdnussöl | 19,8 g |
| | 100,8 g = 100 ml |

Die Wirksubstanz wird in dem Benzylalkohol unter Rühren, eventuell auch unter leichtem Erwärmen, gelöst. Zu der Lösung wird das Natriumdioctylsulfosuccinat und das Erdnussöl gegeben und unter Erwärmen und gründlichem Durchmischen gelöst.

Beispiel 3

Wirkung gegen pflanzenschädigende Akariden: Tetranychus urticae (OP-sensibel) und Tetranychus cinnabarinus (OP-tolerant)

Die Primärblätter von Phaseolus vulgaris Pflanzen werden 16 Stunden vor dem Versuch auf akarizide Wirkung mit einem infestierten Blattstück aus einer Massenzucht von Tetranychus urticae (OP-sensibel) oder Tetranychus cinnabarinus (OP-tolerant) belegt (die Toleranz bezieht sich auf die Verträglichkeit von Diazinon).

Die so behandelten infestierten Pflanzen werden mit einer Versuchslösung, welche 400 oder 200 ppm der zu prüfenden Verbindung enthält, bis zur Tropfnässe besprüht.

Nach 24 Stunden und wiederum nach 7 Tagen werden Imagines und Larven (alle beweglichen Stadien) unter dem Binokular auf lebende und tote Individuen ausgewertet.

Man verwendet pro Konzentration und pro Testspezies eine Pflanze. Während des Versuchsverlaufs stehen die Pflanzen in Gewächshauskabinen bei 25°C.

Die Verbindungen der Formel I zeigen in diesem Versuch eine mindestens 90%ige Abtötung gegen Individuen der Spezies Tetranychus urticae und Tetranychus cinnabarinus.

Beispiel 4

Wirkung gegen ektoparasitäre Akariden: (Zekken) Rhipicephalus bursa (Imagines und Larven), Amblyomma hebraeum (♀ Imagines, Nymphen und Larven) und Boophilus microplus (Imagines, Larven - OP-sensibel und OP-tolerant)

Als Testobjekte werden Larven (jeweils ca. 50), Nymphen (jeweils ca. 25) oder Imagines (jeweils ca. 10) der Zeckenarten Rhipicephalus bursa, Amblyomma hebraeum und Boophilus microplus verwendet. Die Testtiere werden für kurze Zeit in eine wässrige Emulsion bzw. Lösung getaucht, welche die zu prüfende Verbindung in einer geeigneten Konzentration zwischen 0,1 und 1000 ppm enthält.

Die in Teströhrchen befindlichen Emulsionen bzw. Lösungen werden dann mit Watte aufgenommen und die benetzten Testtiere in den so kontaminierten Röhrchen belassen.

Eine Auswertung der erzielten Abtötungsrate bei jeder Konzentration erfolgt für Larven nach 3 Tagen und für Nymphen und Imagines nach 14 Tagen.

Die Verbindungen der Formel I zeigen in diesem Versuch eine mindestens 90%ige Abtötung gegen Larven, Nymphen und Imagines der Spezies Rhipicephalus bursa und Amblyomma hebraeum sowie gegen Imagines und Larven (OPres. und OP-sens.) der Spezies Boophilus microplus. Die Verbindungen Nr. 1a, 2a, 4a, 5a, 9a, 11a, 16a, 17a, 1b, 3b und 7b-10b sind 100%ig wirksam.

**Patentansprüche**

1. Verbindungen der Formel I

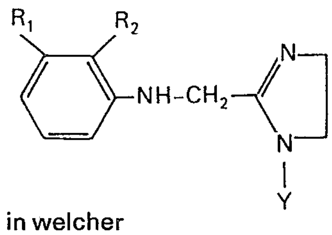

(I)

in welcher

$R_1$ und $R_2$ unabhängig voneinander ein Chloratom oder die Methylgruppe,

Y die Reste 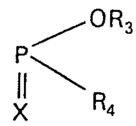 oder $-CH=N-R_5$,

bedeuten und worin

$R_3$ Methyl oder Äthyl,

$R_4$ Alkyl mit 1 bis 4 Kohlenstoffatomen, Alkoxy mit 1 bis 2 Kohlenstoffatomen, Alkylthio mit 1 bis 4 Kohlenstoffatomen oder Phenyl,

X ein Sauerstoffatom oder ein Schwefelatom und

$R_5$ einen mit einem Kohlenstoffatom an den Rest des Moleküls gebundenen unsubstituierten oder substituierten Pyridinylrest, dessen Substituenten aus der Gruppe Halogen und Alkyl mit 1 bis 4 Kohlenstoffatomen ausgewählt sind, darstellen, einschliesslich ihrer Säureadditionssalze.

2. Verbindungen der Formel I gemäss Anspruch 1, in denen $R_1$ und $R_2$ unabhängig voneinander Chlor oder Methyl sind und im Rest Y $R_3$ Äthyl, $R_4$ Alkylthio mit 3 oder 4 Kohlenstoffatomen und X ein Sauerstoffatom bedeuten oder $R_5$ ein in 2-Stellung an den Rest des Moleküls gebundenen unsubstituierten oder methyl-substituierten Pyridinylrest darstellt, einschliesslich ihrer Säureadditionssalze.

3. Eine Verbindung aus der Gruppe:

1-(O-Äthyl-S-sek.butyl-thiophosphonyl-2-(2′,3′-dimethylanilinomethyl)-2-imidazolin,

1-(O-Äthyl-S-isobutyl-thiophosphonyl-2-(2′,3′-dimethylanilinomethyl)-2-imidazolin,

1-(O-Äthyl-S-n-propyl-thiophosphonyl-2-(2′,3′-dimethylanilinomethyl)-2-imidazolin,

1-(O-Äthyl-äthyl-thiophosphonyl-2-(2′,3′-dimethylanilinomethyl)-2-imidazolin,

1-(O-Äthyl-S-isobutylthiophosphonyl-2-(2′,3′-dichloranilinomethyl)-2-imidazolin,

1-(O-Äthyl-S-n-propylthiophosphonyl-2-(2′,3′-dichloranilinomethyl)-2-imidazolin,

1-(O-Äthyl-S-n-propyl-thiophosphonyl-2-(2'-methyl-3'-chloranilinomethyl)-2-imidazolin,
1-(O-Äthyl-S-sek.butyl-thiophosphonyl-2-(2'-methyl-3'-chloranilinomethyl)-2-imidazolin.

4. Eine Verbindung aus der Gruppe:
N-([2-(2,3-Dimethylanilinomethyl)-2-imidazolin-1-yl]-methyliden)-N-(6-methylpyridin-2-yl)imin,
N-([2-(2,3-Dimethylanilinomethyl)-2-imidazolin-1-yl]-methyliden)-N-(pyridin-2-yl)imin,
N([2-(2,3-Dichloranilinomethyl)-2-imidazolin-1-yl]-methyliden)-N-pyridin-2-yl)imin,
N-([2-(2,3-Dichloranilinomethyl)-2-imidazolin-1-yl]-methyliden)-N-(3-methylpyridin-2-yl)imin,
N-([2-(2,3-Dichloranilinomethyl)-2-imidazolin-1-yl]-methyliden)-N-(6-methylpyridin-2-yl)imin
und
N-([2-(2,3-Dichloranilinomethyl)-2-imidazolin-1-yl]-methyliden)-N-(4-methylpyridin-2-yl)imin.

5. Verfahren zur Herstellung von Verbindungen der Formel I

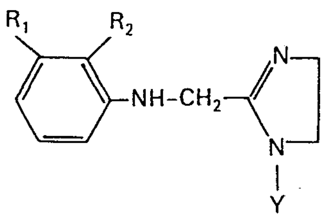

(I)

in welcher
$R_1$ und $R_2$ unabhängig voneinander ein Chloratom oder die Methylgruppe,

Y den Rest 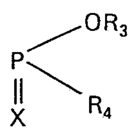 oder den Rest $-CH=N-R_5$

bedeuten und worin
$R_3$ Methyl oder Äthyl,
$R_4$ Alkyl mit 1 bis 4 Kohlenstoffatomen, Alkoxy mit 1 bis 2 Kohlenstoffatomen, Alkylthio mit 1 bis 4 Kohlenstoffatomen oder Phenyl,
X ein Sauerstoffatom oder ein Schwefelatom und
$R_5$ einen mit einem Kohlenstoffatomen an den Rest des Moleküls gebundenen unsubstituierten oder substituierten Pyridinylrest, dessen Substituenten aus der Gruppe Halogen und Alkyl mit 1 bis 4 Kohlenstoffatomen ausgewählt sind, darstellen, dadurch gekennzeichnet, dass man eine Verbindung der Formel II

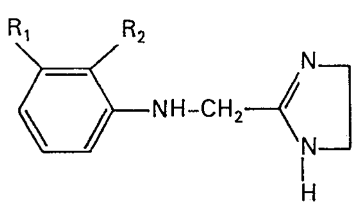

(II)

worin $R_1$ und $R_2$ die unter Formel I angegebenen Bedeutungen haben, entweder
a) mit einer Verbindung der Formel IIIa

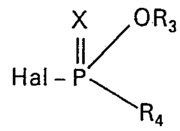

(IIIa)

worin $R_3$, $R_4$ und X die unter Formel I angegebenen Bedeutungen haben und Hal ein Halogenatom, insbesondere ein Chlor- oder Bromatom, darstellt, oder
b) mit einer Verbindung der Formel IIIb

$R_6-O-CH=N-R_5$ (IIIb)

worin $R_5$ die unter Formel I angegebenen Bedeutungen hat und $R_6$ Methyl oder Äthyl darstellt, umsetzt.

6. Schädlingsbekämpfungsmittel, welches neben Träger- und/oder Zusatzstoffen als aktive Komponente eine Verbindung der Formel I gemäss Anspruch 1 oder ein Säureadditionssalz davon, enthält.

7. Verfahren zur Bekämpfung von Schädlingen durch Verwendung einer Verbindung der Formel I gemäss Anspruch 1 oder eines Säureadditionssalz davon.

8. Verfahren gemäss Anspruch 7, wobei die Schädlinge Vertreter der Ordnung Acarina sind.

9. Verfahren gemäss Anspruch 7, wobei die Schädlinge Ektoparasiten sind.

10. Verfahren gemäss Anspruch 7, wobei die Schädlinge Vertreter der Ordnung Phthiraptera und Phytophthires sind.

11. Verfahren gemäss Anspruch 7, wobei die Schädlinge Vertreter der Familie Calliphoridae sind.

**Claims**

1. A compound of the formula I

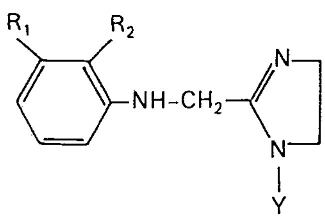

(I)

wherein
$R_1$ and $R_2$ are each independently of the other a chlorine atom or the methyl group,

Y is the group 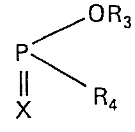

or the group $-CH=N-R_5$,
wherein
$R_3$ is methyl or ethyl,
$R_4$ is alkyl having 1 to 4 carbon atoms, alkoxy having 1 or 2 carbon atoms, alkylthio having 1 to 4 carbon atoms, or phenyl,
X is an oxygen atom or a sulfur atom, and

$R_5$ is an unsubstituted or substituted pyridinyl group which is attached through a carbon atom to the residue of the molecule, the substituents being selected from the group consisting of halogen and alkyl having 1 to 4 carbon atoms, or an acid addition salt thereof.

2. A compound of the formula I according to claim 1, wherein each of $R_1$ and $R_2$ independently of the other is chlorine or methyl, and, in the group Y, $R_3$ is ethyl, $R_4$ is alkylthio having 3 or 4 carbon atoms, and X is an oxygen atom, or $R_5$ is an unsubstituted or methyl-substituted pyridinyl group attached in the 2-position to the residue of the molecule, or an acid addition salt thereof.

3. A compound selected from the group consisting of:
1-(O-ethyl-S-sec-butylthiophosphonyl-2-(2′,3′-dimethylanilinomethyl)-2-imidazoline,
1-(O-ethyl-S-isobutylthiophosphonyl-2-(2′,3′-dimethylanilinomethyl)-2-imidazoline,
1-(O-ethyl-S-n-propylthiophosphonyl-2-(2′,3′-dimethylanilinomethyl)-2-imidazoline,
1-(O-ethylethylthiophosphinyl-2-(2′,3′-diemethylanilinomethyl)-2-imidazoline,
1-(O-ethyl-S-isobutylthiophosphonyl-2-(2′,3′-dichloroanilinomethyl)-2-imidazoline,
1-(O-ethyl-S-n-propylthiophosphonyl-2-(2′,3′-dichloroanilinomethyl)-2-imidazoline,
1-(O-ethyl-S-n-propylthiophosphonyl-2-(2′-methyl-3′-chloroanilinomethyl)-2-imidazoline, and
1-(O-ethyl-S-sec-butylthiophosphonyl-2-(2′-methyl-3′-chloroanilinomethyl)-2-imidazoline.

4. A compound selected from the group consisting of:
N-([2-(2,3-dimethylanilinomethyl)-2-imidazolin-1-yl]-methylidene)-N-(6-methylpyridin-2-yl) imine,
N-([2-(2,3-dimethylanilinomethyl)-2-imidazolin-1-yl]-methylidene)-N-(pyridin-2-yl)imine,
N-([2-(2,3-dichloroanilinomethyl)-2-imidazolin-1-yl]-methylidene)-N-(pyridin-2-yl)imine,
N-([2-(2,3-dichloroanilinomethyl)-2-imidazolin-1-yl]-methylidene)-N-(3-methylpyridin-2-yl) imine,
N-([2-(2,3-dichloroanilinomethyl)-2-imidazolin-1-yl]-methylidene)-N-(6-methylpyridin-2-yl) imine, and
N-([2-(2,3-dichloroanilinomethyl)-2-imidazolin-1-yl]-methylidene)-N-(4-methylpyridin-2-yl) imine.

5. A process for the preparation of a compound of the formula I

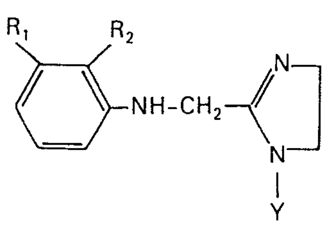

(I)

wherein
$R_1$ and $R_2$ are each independently of the other a chlorine atom or the methyl group,
Y is the group

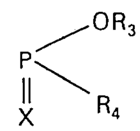

or the group $-CH=N-R_5$, wherein

$R_3$ is methyl or ethyl,
$R_4$ is alkyl having 1 to 4 carbon atoms, alkoxy having 1 or 2 carbon atoms, alkylthio having 1 to 4 carbon atoms, or phenyl,
X is an oxygen atom or a sulfur atom, and
$R_5$ is an unsubstituted or substituted pyridinyl group which is attached through a carbon atom to the residue of the molecule, the substituents being selected from the group consisting of halogen and alkyl having 1 to 4 carbon atoms,
which process comprises reacting a compound of the formula II

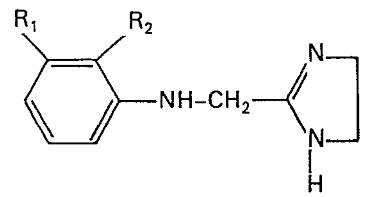

(II)

wherein $R_1$ and $R_2$ are as defined for formula I, either
a) with a compound of the formula IIIa

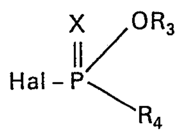

(IIIa)

wherein $R_3$, $R_4$ and X are as defined for formula I, and Hal is a halogen atom, preferably a chlorine or bromine atom; or
b) with a compound of the formula IIIb

$$R_6-O-CH=N-R_5 \qquad \text{(IIIb)}$$

wherein $R_5$ is as defined for formula I, and $R_6$ is methyl or ethyl.

6. A pesticidal composition which contains as active ingredient a compound of the formula I according to claim 1, or an acid addition salt thereof, together with carriers and/or additives.

7. A method of controlling pests at a locus, which method comprises applying to said locus a pesticidally effective amount of a compound of the formula I according to claim 1, or of an acid addition salt thereof.

8. A process according to claim 7, wherein the pests are representatives of the order Acarina.

9. A process according to claim 7, wherein the pests are ectoparasites.

10. A process according to claim 7, wherein the pests are members of the orders Phthiraptera and Phytophthires.

11. A process according to claim 7, wherein the pests are members of the family Calliphoridae.

**Revendications**

1. Composés de formule I

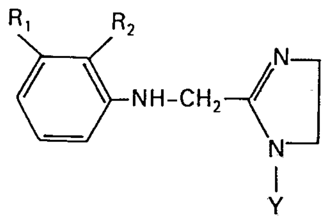

(I)

dans laquelle:

$R_1$ et $R_2$ représentent, indépendamment l'un de l'autre, un atome de chlore ou le groupe méthyle;

Y représente les restes

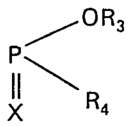

ou $-CH=N-R_5$ dans lesquels

$R_3$ représente un groupe méthyle ou éthyle;
$R_4$ représente un groupe alkyle en $C_1-C_4$, alcoxy en $C_1-C_2$, alkylthio en $C_1-C_4$ ou phényle,
X représente un atome d'oxygène ou de soufre, et
$R_5$ représente un reste pyridinyle non substitué ou substitué et relié au reste de la molécule par l'intermédiaire d'un atome de carbone, et dont les substituants sont choisis dans le groupe formé par les halogènes et les groupes alkyle en $C_1-C_4$,
y compris leurs sels formés par addition avec des acides.

2. Composés de formule I selon la revendication 1, dans lesquels $R_1$ et $R_2$ représentent indépendamment l'un de l'autre, le chlore ou un groupe méthyle, et dans le reste Y $R_3$ représente un groupe éthyle, $R_4$ représente un groupe alkylthio en $C_3$ ou $C_4$ et X représente un atome d'oxygène ou bien $R_5$ représente un reste pyridinyle non substitué ou méthylsubstitué relié au reste de la molécule en position 2, y compris leurs sels formés par addition avec des acides.

3. Un composé du groupe:
1-(O-éthyl-S-sec.butyl-thiophosphonyl-2-(2',3'-diméthylanilinométhyl)-2-imidazoline;
1-(O-éthyl-S-isobutyl-thiophosphonyl-2-(2',3'-diméthylanilinométhyl)-2-imidazoline;
1-(O-éthyl-S-n-propyl-thiophosphonyl-2-(2',3'-diméthylanilinométhyl)-2-imidazoline;
1-(O-éthyl-éthyl-thiophosphonyl-2-(2',3'-diméthylanilinométhyl)-2-imidazoline;
1-(O-éthyl-S-isobutylthiophosphonyl-2-(2',3'-dichloranilinométhyl)-2-imidazoline;
1-(O-éthyl-S-n-propyl-thiophosphonyl-2-(2',3'-dichloranilinométhyl)-2-imidazoline;
1-(O-éthyl-S-n-propyl-thiophosphonyl-2-(2'-méthyl-3'-chloranilinométhyl)-2-imidazoline;
1-(O-éthyl-S-sec.butyl-thiophosphonyl-2-(2'-méthyl-3'-chloranilinométhyl)-2-imidazoline.

4. Un composé du groupe:
N-([2-(2,3-diméthylanilinométhyl)-2-imidazoline-1-yl]-méthylidène)-N-(6-méthylpyridino-2-yl)-imine;
N-([2-(2,3-diméthylanilinométhyl)-2-imidazoline-1-yl]-méthylidène)-N-(pyridino-2-yl)-imine;
N-([2-(2,3-dichloranilinométhyl)-2-imidazoline-1-yl]-méthylidène)-N-(pyridino-2-yl)-imine;
N-([2-(2,3-dichloranilinométhyl)-2-imidazoline-1-yl]-méthylidène)-N-(3-méthylpyridino-2-yl)-imine;
N-([2-(2,3-dichloranilinométhyl)-2-imidazoline-1-yl]-méthylidène)-N-(6-méthylpyridino-2-yl)-imine; et
N-([2-(2,3-dichloranilinométhyl)-2-imidazoline-1-yl]-méthylidène)-N-(4-méthylpyridino-2-yl)-imine.

5. Procédé de préparation des composés de formule I

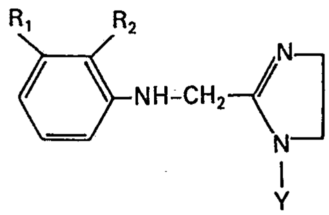

(I)

dans laquelle
$R_1$ et $R_2$ représentent, indépendamment l'un de l'autre, un atome de chlore ou le groupe méthyle;

Y représente le reste lequel:

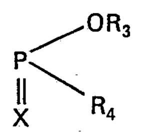

ou le reste $-CH=N-R_5$ dans lequel:

$R_3$ représente un groupe méthyle ou éthyle;
$R_4$ représente un groupe alkyle en $C_1-C_4$, alcoxy en $C_1-C_2$, alkylthio en $C_1-C_4$ ou phényle,
X représente un atome d'oxygène ou de soufre, et
$R_5$ représente un reste pyridinyle non substitué ou substitué relié au reste de la molécule par l'intermédiaire d'un atome de carbone et dont les substituants sont choisis dans le groupe formé par les halogènes et les groupes alkyle en $C_1-C_4$,
caractérisé en ce que l'on fait réagir un composé de formule II

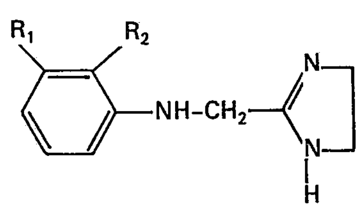

(II)

dans laquelle $R_1$ et $R_2$ ont les significations indiquées en référence à la formule I
a) soit avec un composé de formule IIIa

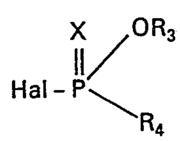

(IIIa)

dans laquelle $R_3$, $R_4$ et X ont les significations indiquées en référence à la formule I et Hal représente un atome d'halogène, en particulier un atome de chlore ou de brome,

b) soit avec un composé de formule IIIb

$$R_6\text{-O-CH=N-}R_5 \qquad \text{(IIIb)}$$

dans laquelle $R_5$ a les significations indiquées en référence à la formule I et $R_6$ représente un groupe méthyle ou éthyle.

6. Produits pesticides contenant, avec des véhicules et/ou additifs, un composé de formule I selon la revendication 1 ou un sel d'un tel composé formé par addition avec un acide en tant que composant actif.

7. Procédé pour combattre les parasites par utilisation d'un composé de formule I selon la revendication 1 ou d'un sel d'un tel composé formé par addition avec un acide.

8. Procédé selon la revendication 7, dans lequel les parasites sont des représentants de l'ordre des acariens.

9. Procédé selon la revendication 7, dans lequel les parasites sont des ectoparasites.

10. Procédé selon la revendication 7, dans lequel les parasites sont des représentants de l'ordre des phthiraptères et des phytophthires.

11. Procédé selon la revendication 7, dans lequel les parasites sont des représentants de la famille des calliphoridés.